# EUROPEAN PATENT SPECIFICATION

(11) **EP 0 712 307 B1**
(45) Date of publication and mention of the grant of the patent: **14.11.2001**
(21) Application number: 94925178.9
(22) Date of filing: 02.08.1994
(51) Int. Cl.: A61J 1/00, A61K 38/16, A61K 39/00, C07K 16/30, C07K 1/00

(54) **METHODS FOR B-CELL POPULATION CONTROL**
VERFAHREN ZUR KONTROLLE VON B-ZELLENPOPULATION
PROCEDES DE REGULATION DE LA POPULATION DES LYMPHOCYTES B

(30) Priority: 02.08.1993 US 101436
(43) Date of publication of application: 22.05.1996
(73) Proprietor: THE BOARD OF TRUSTEES OF THE LELAND STANFORD JUNIOR UNIVERSITY, Palo Alto, CA 94304-1850 (US)
(72) Inventor: BHAT, Neelima, M., Cupertino, CA 95014 (US); BIEBER, Marcia, M., Los Altos, CA 94022 (US); TENG, Nelson, N., H., Hillsborough, CA 94010 (US)
(74) Representative: Greaves, Carol Pauline
(86) International application number: PCT/US94/08793
(87) International publication number: WO 95/03770

(56) References cited:
- US-A- 4 804 626
- US-A- 4 904 481
- THE JOURNAL OF IMMUNOLOGY, Vol. 151, No. 9, issued 01 November 1993, N.M. BHAT et al.: "Human antilipid monoclonal antibodies bind to human B-cells and the i antigen on cord red blood cells", pages 5011-5021, entire document.
- PROC. NATL. ACAD. SCI. USA, Vol. 82, issued 19 March 1985, N.N.H. TENG et al.: "Protection against gram-negative bacteremia and endotoxemia with human monoclonal IGM antibodies", pages 1790-1794, entire document.
- J.W. GODING, "Monoclonal antibodies: Principles and practice", published 1986 by ACADEMIC PRESS (London), pages 219-240, see entire document.

## Description

### Technical Field

The field of this invention is the control of B-cell proliferation in a mammalian host as a therapy.

### Background

The immune system is the first line of defense against many pathologies. Particularly, the lymphoid compartment is concerned with monitoring tumorigenesis. invasion by pathogens, such as bacteria and viruses, aiding in the removal of foreign bodies, and the like. Essential to the ability of the lymphoid compartment to protect the host against the various pathologies is the ability to recognize self from non-self. In monitoring tumorigenesis, subtle distinctions may be involved and the high incidence of cancer, particularly in the aged, suggests that the monitoring frequently breaks down over time. In addition, because of the enormous diversity of the environment to which the immune system is exposed, there is always the possibility that epitopes will be encountered, which may trigger an immune response which can be directed against self. Other mechanisms may also be operative in the process where a lymphoid cell attacks an endogenous epitope. These autoimmune diseases can be extremely destructive, as is evidenced by diabetes, rheumatoid arthritis, neuronal diseases, such as multiple sclerosis, and the like. While in many cases, the disease is associated with T-cell attack, in some of the diseases, there may be a B-cell component, and in other diseases, such as rheumatoid arthritis and lupus nephritis, the primary mediator may be B-cells.

The lymphoid compartment may be more susceptible than other cells to tumorigenesis, because of the recombinatorial processes associated with the rearrangements involved with formation of immunoglobulins and the T-cell receptor. Lymphoid cancers, such as lymphomas and leukemias are particularly dangerous, because of the opportunity for migration of the lymphoid cells throughout the body and the many sites in the periphery, where lymphocytes reside, so as to provide numerous opportunities for metastasis. Furthermore, these diseases interfere with the native process which is intended to monitor tumorigenesis.

There is, therefore, substantial interest in being able to develop techniques and therapies which will allow for selective reduction in cell types associated with pathogenesis.

### Relevant Literature

Grillot-Courvalin et al. (1992) *Eur*. *J. Immunol*. *22:1781,* describe an anti-B cell autoantibody from Wiskott-Aldrich syndrome which recognizes i blood group specificity on normal human B-cells. The production of human monoclonal antibodies is described by Bieber and Teng (1987) *"In vitro* sensitization for the production of human monoclonal antibodies," in *Human Hybridomas,* A.J. Strelkauskas ed. Marcel Dekker, Inc., New York, p. 39. Kannagi et al. (1983) *Cancer Res. 43:4997*, describe factors affecting expression of glycolipid tumor antigens. Niemann et al. (1978) *Biochem. Biophys. Res. Comm.* 81:1286, describe Blood group i and I activities of "lacto-N-nor-hexaosylceramide" and its analogues, particularly the structural requirements for i specificities Teng et al. ((1985) *Proc*. *Natl*. *Acad*. *Sci*. *USA* **82**:1790) discloses the production of human monoclonal IgM antibodies against bacterial lipopolysaccharide ("LPS") and demonstrated that monoclonal antibody A6H4C5 was directed against covalently bound lipid A, the most conserved structure of LPS amongst Gram-negative bacteria.

### SUMMARY OF THE INVENTION

Methods are provided for inducing cell death in B-cells, including neoplastic B-cells, by employing reagents that bind to a B-cell marker. Particularly, antibodies specific for the marker can be administered to a host to induce death in B-cells to which the antibodies bind or can be used in *ex vivo* clinical situations to selectively remove B-cells.

According to a first aspect of the present invention there is provided a polyvalent agent that binds more than one CDIM epitope, for the manufacture of a medicament for treatment of B cell proliferative disease.

According to a second aspect of the present invention there is provided a method for killing B cells within a mixed population of cells *in vitro,* said method comprising contacting said mixed population of cells with a cytotoxic amount of a polyvalent agent that binds more than one CDIM epitope.

According to a third aspect of the present invention there is provided a method of isolating a B-cell marker molecule, wherein the marker is characterized by having a CDIM epitope, is surface accessible on B cells, and is recognized by MAb 216 (the monoclonal antibody secreted by hybridoma cell line ATCC deposit No. HB 11659), said method comprising combining a lysate, made from a cell population containing B cells, with an antibody binding to the CDIM epitope to form an immune complex; separating said immune complex from other components of said lysate; and releasing the B-cell marker molecule from said complex with a compound competitive for binding to said antibody.

According to a fourth aspect of the present invention there is provided the monoclonal antibody secreted by hybridoma cell line ATCC deposit No. HB 11659.

According to a fifth aspect of the present invention there is provided the hybridoma cell line ATCC deposit No. HB 11659.

In accordance with the subject invention, methods and compositions are provided for killing B-cells, particularly neoplastic B-cells, in cellular compositions comprising a plurality of cells, particularly hematopoietic cells. This allows for a therapy in the treatment of the aberrant proliferation of B-cells and for the selective removal of B-cells from cultures or other *ex vivo* situations. By B-cells is intended those cells of the B-cell lineage, where B-cells may be defined as comprising surface membrane protein markers found on normal B-cells, such as CD19, CD20, CD21 and CD22.

The CDIM epitope is a three-dimensional structural conformation recognized on normal human, peripheral, and splenic B-cells and on some neoplastic B-cells by the human monoclonal antibody 216. The epitope is defined structurally in terms of spatial conformation, functionally in terms of specific antibody binding, and cytologically in terms of cellular distribution, as described below.

The spatial conformation of CDIM is characterized by being a hexa- or longer, straight-chain oligosaccharide comprising acyl-substituted repeating glucosamine subunits. The epitope is structurally related to the "I" and "i" antigens present on adult and cord red blood cells (RBC's) respectively. Structurally related synthetic sugars include lacto-N-norhexaosyl ceramide. The CDIM epitope can be identified on a B-cell surface using fluorescent-labelled human monoclonal antibody (MAb), particularly the human monoclonal antibody 216. Cells carrying the epitope can be analyzed, for example, by a fluorescence-activated cell sorter (FACS). For example, a human MAb can be biotin labelled and detected with fluorescent-labelled streptavidin, where control human MAbs do not bind to the human B-cells.

The CDIM epitope is naturally presented as a glyco-moiety found on substantially all peripheral B-lymphocytes and splenic B-lymphocytes and on certain cultured B-cell lymphoma lines, such as Lam, REH, and JY25. It is also found on 30-40% of primary B-cell lymphomas of various histopathologic classifications. At least about 90% of these various categories of cells, more usually about 100% of these cells, will present the indicated epitope. The human monoclonal antibody 216, which recognizes CDIM on B-cells as described above, does not recognize this epitope on cultured T-cells such as Peer and HUT 78, macrophage lines such as U937, since the CDIM epitope is not present on normal T cells, macrophages, NK cells, epithelial, endothelial or mesenchymal cells.

The present inventors first discovered that the CDIM epitope exists on B-cells, that certain human polyreactive natural MAbs can bind to the CDIM ligand on human B lymphocytes, and that the binding of at least two CDIM epitopes on the same B-cell leads to B-cell death. As demonstrated in the Examples section, human monoclonal antibodies designated MAb 216 and MAb A6H4C5 are among those that the have been discovered to bind to the CDIM epitope and lead to B-cell death.

The ligand recognized by 216 and A6H4C5 on human B lymphocytes is cleaved by the enzyme endo-β-galactosidase, indicating it is a carbohydrate antigen structurally related to the polylactosamine chain of i Ag. Grillot-Courvalin et al. ((1992) *Eur. J. Immunol.* 22:1781) have reported human MAbs (HY18 and AY21) derived from a single patient with Wiskott-Aldrich syndrome, which react with a human B cell subset and have cold agglutinin specificity for the i Ag of cord RBC. Unlike the HY18 and HY21 antibodies that react to only 60% of the CD19⁺ cells in human tonsils, spleen, and peripheral blood, MAbs 216 and A6H4C5 bind all CD19⁺ and CD20⁺ B lymphocytes in human peripheral blood and spleen, MAbs 216 and A6H4C5 do not distinguish B cells by the isotope expressed, binding IgG⁺ and IgM⁺ cells with equal intensity, and also bind all B cells regardless of their CD5 expression. A6H4C5 and 216 also react to murine splenic and peritoneal B220⁺ cells. The ligand recognized on B lymphocytes is apparently conserved among different species. It follows that the B-cell CDIM-binding molecule ("receptor") of the invention find use in diagnostics, particularly for methods that require B-cell detection, identification, number or percentage. Useful in diagnostics are kits that contain B-cell CDIM-binding molecules of the invention. Using methods known in the art, the CDIM-binding molecules of the invention can be modified to contain detectable markers suitable for allowing detection of B-cell bound CDIM-binding molecules of the invention.

For the purpose of this invention polyvalent (two or more binding sites) CDIM-binding receptors the required. By "receptor" is intended a compound which has a specific affinity for are CDIM epitope, generally at least about 10⁻⁷ M, preferably at least about 10⁻⁸ M, so as to be able to bind the B-cell marker. The polyvalent nature of the receptor allows the simultaneous binding of at least two CDIM B-cell marker molecules on the cell membrane surface, thereby forming a cross-link. Conveniently, antibodies can be used from any of the immunoglobulin families, such as A, D, E, G, and M; it is not requisite that the antibody be associated with various cytotoxic processes associated with particularly Fc-initiated processes. Usually, the antibody will be IgM, since the pentameric structure of this molecule allows cross-linking unhindered by steric interference. Binding of at least two CDIM marker molecules on the same cell surface by the same receptor results ultimately in cell death. Besides antibodies, other receptors with the indicated affinity will find use, where the receptor can, for example, be associated with a lectin either naturally occurring or modified. Alternatively, small synthetic molecules can be devised which will allow for specific binding and cross linking of the CDIM epitope. In addition, one may subject the variable region of a MAb to mutagenesis to enhance the binding affinity of an antibody for the CDIM epitope, if desired. Combinatorial libraries, such as bacteriophage libraries displaying human Ig repertoires, employed with the teachings herein provide a diversity of polyvalent agents for screening to identify additional CDIM-binding agents. A portion of a CDIM-binding antibody of the invention that retains B-cell binding function can be conjugated to a cytotoxic molecule, such as a peptide toxin (e.g., pseudomonas exotoxin, ricin A chain), using methods known in the art to create new macromolecules capable of B-cell killing. A Fab portion of a MAb of the invention is preferred for conjugation. A particularly preferred conjugate is the fusion of the Mab 216 Fab2 portion with a pseudomonas exotoxin. Cytotoxic conjugates can be created using means known in the art including chemical conjugation, such as site-specific conjugation, and DNA gene fusion technology, so long as the conjugate retains B-cell binding specificity and has B-cell toxicity.

Using the screening methods as taught herein other MAbs or polyvalent agents with ability to kill human B cells can be identified. Preferred antibodies for screening are those whose heavy chain region are encoded by the VH4.21 gene. MAbs for screening, from which MAbs of the invention are identified, can be obtained from hybridomas generated from B cells of hosts immunized with an antigen displaying the CDIM epitope or a structure with similar spatial conformation or from B cells stimulated with LPS or other cross-reactive antigen prior to fusion either by host immunization (Teng et al. 1985) or by *in vitro* sensitization of B-cells (see Examples; Bieber and Teng (1987) *"In vitro* sensitization for the production of human monoclonal antibodies, " in *Human Hybridomas*, A.J. Strelkauskas ed. Marcel Dekker, Inc., New York, p. 39). Monoclonal antibodies useful in the invention can also be obtained by (1) fusing a heteromyeloma to EBV transformed B cells from normal peripheral blood or spleen, (2) selecting hybridomas that present antibodies that are IgM and 9G4 positive (9G4 is a rat anti idiotype Ab that is known in the art to specifically recognize VH4.21 protein chains in the framework region of an antibody (available commercially from F. K. Stevenson, Southampton University, S09 4XY, England)), (3) screening, by FACS for example, for monoclonals that bind to human B-cells, and (4) selecting those B-cell positives that bind to synthetic CDIM epitope as taught herein. All cytotoxic monoclonal antibodies are cold agglutinins with specificity for the i antigen of cord red blood cells, preferably "high titer" anti i, i.e. having an end point in the nanogram/ml range. The i antigen is the straight chain lactosamine as opposed to the branched chain of the I antigen.

The CDIM-binding agents can be used in therapy for treatment of B-cell proliferative diseases, such as B-cell neoplasia, systemic lupus erythematosus ("SLE"), and autoimmune diseases. Thus, the subject agents will find application in the treatment of autoimmune-mediated disease, particularly B-cell-mediated disease. And particular in rheumatoid arthritis and lupus nephritis. For example, the human MAb 216, by binding to at least two CDIM epitopes on the same cell, causes the death of the cell expressing this epitope. Epitope-mediated death does not require complement or cell-mediated lysis. While, for the most part, human cells in human patients will be a primary interest, other animals, particularly domestic animals, will also be served by the subject methodology to the extent that a given agent reacts across species, which is readily determined by binding studies of the type described herein. It follows that injection into a host of an effective dose of B-cell cytotoxic molecules of the invention that kills all peritoneal B cells can be used to prepare B cell free compositions. This method is particularly advantageous for use with species where preparation of B-cell free compositions by conventional *in vitro* methods is time-consuming or inefficient. Mice are a preferred host for application of this method. MAbs 216 and A6H4C5 are preferred for use in this method.

For therapeutic uses, the compositions and selective agents disclosed herein can be administered by any convenient technique, which can vary depending on the nature of the compound/agent, the purpose and frequency of the treatment, and the like. For small molecular weight agents, oral administration is preferred, and enteric coatings are indicated where the compound is not expected to retain activity after exposure to the stomach environment. Generally the amount administered will be empirically determined, typically in the range of about 0.1 to 1000 *µ*g active ingredient per kg of recipient, with adjustment by a physician or other person after consideration of clinical results. For administration of larger molecules such as antibodies, generally larger doses are needed typically in the range of about 1 to 10 mg per kg of host.

Large proteins are preferably administered parenterally or systemically, conveniently in a physiologically acceptable carrier, e.g., phosphate buffered saline, saline, or deionized water. Some agents such as antibodies can also be administered nasally. Typically, compositions are administered to a retained physiological fluid such as blood. Other additives can be included, such as stabilizers, or bactericides. These additives, if present, will be present in conventional amounts.

The subject agents can also be used for treating cell populations in culture to diminish the B-cell population, whether normal or neoplastic, in the culture. Thus, in mixed cultures, where one wishes to avoid interference by B-cells, where one is interested in studying antigen-presenting-cell mechanisms other than those associated with B-cells, where one is analyzing for cells associated with mediating secretion of a particular cytokine, or where one wishes to study a mixed cell population for other purposes without the presence of B-cells, this can be achieved by adding an amount of the subject agent effective to remove substantially all of the B-cells present in the culture. In a similar manner *ex vivo* therapeutic treatments can be utilized in which blood is removed from a patient into an external environment (as in dialysis), treated to remove excess B-cells, and then returned to the patient. CDIM-binding molecules of the invention can be attached to solid surfaces for use in B-cell binding as a means to isolate or remove B-cells from a mixed cell population.

Where CDIM epitope-specific antibodies are administered therapeutically, it is desirable to minimize the likelihood of an immunogenic or allergenic response by using host-specific antibodies (e.g., human antibodies in humans). While intact antibodies are commonly used, the antibodies may be modified in a variety of ways, by enzymatic cleavage to provide fragments, reduction of disulfide linkages, and the like.

In referring to an isolated component or compound, the isolated component or compound will constitute at least about 1%, usually at least about 10%, and more usually at least about 50% by weight of the isolated material. By pure compound or composition is intended at least about 90%, usually at least 95%, and more usually at least about 99% by weight of the component or compound. Unless otherwise indicated, functional fragments will also be intended when referring to components or compounds.

The following examples are offered by way of illustration and not by way of limitation.

### EXAMPLES

### Production, characterization, and conjugation of human MAbs.

Human MAb 216 was prepared by fusion of uninvolved spleen lymphocytes from a patient with nodular lymphoma. The cells were incubated *in vitro* with LPS and fused to the heteromyeloma line SHMD33. This antibody was found to be mu, lambda using peroxidase-labeled chain-specific antibodies (Cal Tag, South San Francisco, CA). Nucleotide analysis of the heavy chain V region revealed it was encoded by the VH 4.21 gene.

The human MAb A6H4C5 was prepared by fusion of the heteromyeloma SHMA6 with EBV-transformed lymphocytes from human spleen of a Hodgkin's disease patient immunized with the J5 mutant of *Escherichia coli* 0111-B4 (a mutant lacking the O-specific side chain) (Teng et al. 1985). The antibody was found to be *µ* κ by ELISA using peroxidase-labeled chain-specific antibodies (Cal Tag, South San Francisco). As discovered by the present inventors MAb A6H4C5 binds to human B-cells and leads to B-cell death.

Human MAbs were purified on high pressure liquid chromatography using a carboxymethyl column (BioRad, Richmond, CA). Hybridoma supernatant containing 1% FCS was diluted 1:4 with 20 mM Na acetate pH 5.5 The MAbs were eluted with 300 mM NaCl Tris buffer pH 8, dialyzed in PBS and concentrated if necessary on a Centriprep concentrator (Amicon, Danvers, MA). By PAGE analysis the purified material was 85-90% IgM and also contained transferrin and BSA. Concentration of the purified immunoglobulins was determined by sandwich ELISA using a human polyclonal IgM standard (Cooper Biomedical, Malvern, PA).

MAb 216 and other human IgM MAbs were biotinylated using N-hydroxysuccinimidobiotin (Pierce, Rockford, IL) at a ratio of 60 *µ*g/mg IgM. A6H4C5 was biotinylated using biotin HPDP (N-{6-(biotinamido)hexyl}-3'-(2'-pyridyldithio) propionamide) (Pierce), a sulfhydryl *reactive* reagent following the manufacturer's protocol. Because A6H4C5 partially precipitates at pH > 8, N-hydroxysuccinimidobiotin could not be used.

Using screening methods as taught herein other MAb with ability to kill human B cells can be identified. Additional MAb with ability to loll human B cells were identified including MAb TH, a *µ*λ Mab (derived from a B cell lymphoma; MAb obtained from F. Hsu of Stanford University) and MAb FS3, a *µ*λ MAb (derived from a patient with cold agglutinin disease; obtained from F. Stevenson, Southampton, UK). Sequence analysis revealed that all, including MAb 216 and A6H4C5, were VH4.21 and IgM.

### Flow cytometry

Human adult splenic mononuclear cells were obtained from patients undergoing therapeutic splenectomy, and peripheral blood was obtained from normal volunteers. Lymphoma cells were obtained by biopsy or laparotomy for removal of tumor. All procedures had the approval of the Committee for the Protection of Human Subjects at Stanford University. Spleens were gently teased apart in HBSS with 1% FCS and 0.2% DNase and passed through sterile nylon membranes to obtain single-cell suspensions. Peripheral blood and splenocytes were centrifuged at 800 g for 30 min through a ficol/hypaque gradient (Histopaque-1077, Sigma, St. Louis, MO). The mononuclear cell population was washed three times in HBSS with 1% FCS, and resuspended in staining medium (RPMI with 3 % FCS, 1 mM EDTA, and 0.01 M HEPES)at 2.5 x 10⁷ cells/mi.

Tumor tissue that had been removed from patients at surgery was disassociated into a cell suspension and frozen in DMSO with storage in liquid nitrogen. The cells were thawed and incubated overnight at 37 degrees before staining. The thawed cells were also incubated 24 hours with human MAb 216 or control human IgM MAbs and stained with propidium iodide (PI) which measures cell death.

Multi-parameter flow cytometric analysis (FACS) has been described in detail (Parks et al. (1986) *The Handbook of Experimental Immunology*, *supra*, p. 29). Fluorescent-labeled mouse MAbs against CD epitopes were from Becton Dickinson. 5 x 10⁵ cells were suspended with predetermined saturating concentrations of each of the conjugated fluorescent antibodies in a final volume of 125 *µ*l, and incubated on ice for 15 min. The cells were washed and resuspended in 200 *µ*l of staining medium and analyzed on a highly modified dual-laser FACS II (Becton Dickinson, Mountain View, CA), interfaced with a VAX 6300 computer (Digital Equipment, Maynard, MA) running FACS/desk software (Moore and Kautz (1986) *The Handbook of Experimental Immunology*. *supra* p. 30). Dead cells are identified with the propidium iodide (1 *µ*g/ml) signal collected in the APC- or TR-channel in experiments with three-colors (Parks et al. (1986) *supra*).

### Endo-β-galactosidase treatment of cells

Peripheral blood B lymphocytes were incubated at 37°C for one hour at 5 x 10⁶ cells/ml in Iscove's with 5% FCS, with 15 mU/ml of endo-β-galactosidase (Boehringer Mannheim, Indianapolis, IN). The cells were washed and stained for FACS analysis. Cord RBCs at 50% concentration were incubated with 0.1 U/ml of endo-β-galactosidase at 37°C for 4 hours, washed and tested for hemagglutination.

### RESULTS

### 216 MAb reacts with a carbohydrate ligand on human splenic and peripheral B lymphocytes.

Multiparameter FACS analysis of human mononuclear cells demonstrated that the MAb binds specifically to all B lymphocytes (CD20⁺) obtained from human spleen and adult peripheral blood. MS2B6, a human monoclonal IgM used as an isotype control, did not bind human B lymphocytes, nor did other poly-reactive natural antibodies.

The B lymphocytes reacting with 216 were also positive for other pan-B cell markers, such as CD19, CD21, and CD22. Excess amount (10X) of antibodies to CD19, CD20, CD21, CD22, and IgM did not inhibit the binding of 216 to B cells. 216 does not distinguish between subsets of B lymphocytes, reacting with both CD5⁺ and CD5⁻ B cells. The MAb also did not distinguish between the isotope expressed, reacting with both surface IgG or IgM bearing B lymphocytes. *.

Mononuclear cells from human peripheral blood were treated with endo-β-galactosidase, and then stained with MAb 216. Reactivity to human B lymphocytes is significantly reduced in enzyme-treated cells. Expression of an unrelated B cell marker (CD19) does not change following enzyme treatment. Thus, sensitivity of both B lymphocytes and cord RBC to endo-β-galactosidase treatment, suggests that the epitope recognized by the two antibody on B lymphocytes is also a carbohydrate antigen similar to the linear polylactosamine structure of the "i" antigen.

### 216 MAb binds to lymphoma cells

Twenty-seven primary lymphomas were analyzed by FACS. Twenty-three were B-cell lymphomas. The MAb 216 did not react with any T-cell lymphomas and stained 10 of 23 B-cell lymphoma. The MAb 216 did not stain any small cleaved cell (follicular) lymphoma. The MAb 216 stained the following classes of lymphoma; immunoblastic, diffuse large well differentiated, diffuse large cell, diffuse mixed, and diffuse small cell.

### In vitro B-cell toxicity of 216

Human MAb 216 is incubated in vitro at an Ab concentration of 10-20 *µ*l/ml with various types of cells. The cells are in tissue culture media with heat-inactivated normal human serum or in serum-free media and are incubated at 37°C in 5% CO₂. After 18-24 hours incubation the cells are stained with propidium iodide (PI) (and other Ab for determining type of cell) and analyzed on FACS. Cell death is determined by uptake of PI. When human spleen or peripheral blood lymphocytes are incubated with MAb 216, 60-80% of B cells are killed. Four primary lymphoma cell suspensions that stained with the MAb 216, and two that did not, were incubated 24 hours with either 20 *µ*g/ml of the 216 MAb or control human MAb in medial at 37 degrees in 5 % CO2. The cells were analyzed for cell death using propidium iodide (PI) on FACS. The lymphoma cell suspensions that bound the MAb 216 showed significant PI uptake compared to the control MAb. The two lymphoma cell suspensions that did not bind MAb 216 did not take up PI. When B cell lymphoma lines are incubated with MAb 216, 60-90% of the cells are killed. MAb 216 does not kill T cells, NK cells or monocytes. Other control human MAbs cause 0-5% cell death under the same conditions.

MAbs were incubated in vitro in serum free media with human spleen lymphocytes or B-cell lymphoma lines, and dead cells were determined by FACS analysis with PI. (Cell lines treated with 216 were sorted for PI positive and negative cells on FACS to confirm that the PI+ cells were dead.) Killing was maximal at 16-18 hours and 30-40 *µ*g/ml MAb 216 for 3x10⁶ spleen lymphocytes. Using spleen B cells 70-90% of B cells were killed. Variation in percent killed was observed to vary with the individual spleen. MAb TH and MAb A6H4C5 killed the same percent of B cells at the same concentration of antibody as MAb 216. MAb FS3 killed one-fourth to one-third less cells at the same concentration as 216. MAbs A6H4C5 and TH killed B-cell lymphoma cell (4 different lymphoma cell lines were assayed) with the same efficiency as MAb 216; Mab FS3 was less efficient. In the presence of human complement *in vitro* all four MAbs killed 90-100% of B-cells.

### Isolation of a B-cell Marker Molecule Having the CDIM Epitope

The B cell membrane glycolipids, proteins and glycoproteins, including the B-cell marker molecule having the CDIM epitope, can be extracted from the B cell membrane using techniques known in the art such as solubilization using a non-ionic detergent (such as Triton X). The B-cell membrane solution can be passed over an affinity matrix, such as a column, to which the MAb 216 has been covalently bound, for example, by using N-hydroxy succinimide coupled to acrylamide beads (Affi Gel). The CDIM epitope will bind to the MAb 216 resulting in the B-cell marker molecule having the CDIM epitope being removed from the membrane solution. The column is then washed to remove non-specifically bound proteins or molecules. The B-cell marker molecule having the CDIM epitope can be released from the column by competition with a compound such as lacto-N-nor-hexaosylceramide, contained in liposomes, which will competitive bind to the MAb 216.

### Characterization of CDIM Antigen

### The MAb 216 binds to synthetic I/i antigen.

The following glycolipids were run on TLC: i antigen (lacto-N-nothexaosylceramide), sialyl i antigen, paragloboside (lacto-N-tetraocylceramide), sialyl paragloboside, I antigen (branched), and GM3. The MAb 216 was applied to the plate. The plate was washed and I¹²⁵ -labeled goat anti-human IgM was applied to the plate, washed and incubated with X ray film. The TLC plate was then sprayed with sulfuric acid and heated to visualize the glycolipids. Comparing the TLC plate and exposed film revealed that 216 bound i antigen, sialyl i antigen, I antigen, and sialyl I antigen only. i Antigen (Lacto-N-nor-hexaosylceramide): Galβ 14 GlcNAcβ 1-3 Galβ 1-4 GlcNAcβ 1-3 Galβ 1-4 Glcβ 1-Ceramide. Sialyl I Antigen: NeuAcα 2-3 Galβ 1-4 GlcNAcβ 1-3 Galβ 1-4 GlcNAcβ 1-3 Galβ 1-4 Glcβ 1-Ceramide. Paragloboside (Lacto-N-tetraocylceramide): Galβ 1-4 GlcNAcβ 1-3 Galβ 1-4 Glcβ 1-Ceramide. Sialyl Paragloboside: NeuAc α 2-3 Galβ 1-4 GlcNAcβ 1-3 Galβ 1-4 Glcβ 1- Ceramide. GM3: NeuAc α 2-3 Galβ 1-4 Glcβ 1-Ceramide.

### In Vivo B-Cell Killing

Twenty *µ*g of either Mab 216 or A6H4C5 when injected IP in normal mice killed all the peritoneal B cells within 18 hours. A B-cell free composition was obtained from the injected mice.

### In Vivo Inhibition of Neoplastic B Cell Growth

The *in vivo* inhibition of growth of the human neoplastic B cell line LM3M by an antibody of the invention was demonstrated using death as an endpoint in Balb SCID ("severe combined immunodeficiency") mice. SCID mice, challenged with lymphoma cell lines, have been used extensively to test therapies for lymphoma, particularly human lymphoma. 1x10⁵ LM3M tumor cells were injected intraperitoneally (IP) into SCID mice. Three days after lymphoma cell line injection six test mice received 100 *µ*g MAb 216 IP using a 25 gauge needle in 300 *µ*l of solution, and six control mice received 100 *µ*g MAb MS2B6 (a human IgM monoclonal antibody used as a control) IP using the same conditions as for the test mice. MAb MS2B6 is a human IgM lambda monoclonal antibody, which reacts with the intracellular nuclear matrix, and does not react with cell membrane or kill cells. Mice were observed daily until death. The amount of MAb injected in the mice was equivalent on a weight basis to a 70 kg human receiving 350 milligrams of MAb.

Mice receiving MAb 216 died 46, 53, 55, 55, 62, and 62 days after injection of the human LM3M tumor cells. The mouse that died after 46 days had an abnormal presentation displayed as a small (0.5 cm) tumor located on the outside abdominal wall. The mean survival time of mice receiving MAb 216 was 55.5 ± 6 days and was 57.4 ± 4.2 when the mouse with the abnormal presentation was excluded. Mice receiving control MAb MS2B6 died 45, 46, 49, 49, 49, and 52 days after injection of the human LM3M tumor cells. The mean survival time of mice receiving MAb MS2B6 was 48.3 ± 2.5 days. Survival times were significantly different as analyzed for significance using the t test. Degrees of freedom were 5 and the probability of significance was .0038 (P > .005). Administration of MAb 216 inhibits the growth of B cell lymphoma as indicated by the delayed onset of death in the mice treated with MAb 216. Even a single dose of a B-cell cytotoxic antibody resulted in a significant increase in survival times.

### SUMMARY

216 binds all CD19⁺ and CD20⁺ B lymphocytes in human peripheral blood and spleen. Furthermore, 216 does not distinguish B cells by the isotype expressed, binding IgG⁺ and IgM⁺ cells with equal intensity, and also bind all B cells regardless of their CD5 expression. Accordingly, the ligand being recognized on B lymphocytes is a novel marker, with no apparent similarities to any of the known pan-B cells markers.

It is evident from the above results and disclosure that a novel B-cell marker and a specific oligosaccharide epitope thereof have been identified. In addition, proteins which specifically bind the disclosed epitope are provided. These products and products derivable therefrom find use in diagnosis and therapy.

A deposit of the hybridoma cell line secreting MAb 216 was made with the ATCC in Rockville, Maryland, USA, as Deposit No. HB 11659, on June 14, 1994.

## Claims

1. Use of a polyvalent agent that binds more than one CDIM epitope, which is an epitope recognised by Mab 216 (the monoclonal antibody secreted by hybridoma cell line ATCC Deposit No. HB 11659) for the manufacture of a medicament for treatment of B cell proliferative disease.

2. A method for killing B cells within a mixed population of cells *in vitro,* said method comprising:
contacting said mixed population of cells with a cytotoxic amount of a polyvalent agent that binds more than one CDIM epitope.

3. A method according to claim 2, wherein said polyvalent agent is an antibody.

4. A method according to claim 3, wherein said antibody is a monoclonal antibody.

5. A method according to claim 4, wherein said monoclonal antibody is a human antibody.

6. A method according to claim 4, wherein said monoclonal antibody is an IgM.

7. A method according to claim 2, wherein said B cells are neoplastic.

8. A method according to claim 2, wherein said mixed population of cells is hematopoietic

9. The use according to claim 1, wherein said B cells are neoplastic.

10. The use according to claim 1, wherein said B cells mediate an autoimmune disease.

11. The use according to claim 1, wherein said agent is a monoclonal antibody.

12. The use according to claim 11, wherein said antibody is a human IgM.

13. A method of isolating a B-cell marker molecule, wherein the marker is **characterized by** having a CDIM epitope, is surface accessible on B cells, and is recognized by MAb 216 (the monoclonal antibody secreted by hybridoma cell line ATCC deposit No. HB 11659), said method comprising:
combining a lysate, made from a cell population containing B cells, with an antibody binding to the CDIM epitope to form an immune complex;
separating said immune complex from other components of said lysate; and
releasing the B-cell marker molecule from said complex with a compound competitive for binding to said antibody.

14. The method according to claim 13, wherein said competitive binding compound is lacto-N-norhexaosylceramide.

15. The monoclonal antibody secreted by hybridoma cell line ATCC deposit No. HB 11659.

16. The hybridoma cell line ATCC deposit No. HB 11659.

17. The method of claim 5, wherein the monoclonal antibody is the monoclonal antibody secreted by hybridoma cell line ATCC deposit No. HB 11659.

18. A diagnostic kit comprising the monoclonal antibody secreted by hybridoma cell line ATCC deposit No. HB 11659.

## Patentansprüche

1. Verwendung eines mehrwertigen Mittels, das mehr als ein CDIM-Epitop bindet, das ein Epitop ist, das von Mab 216 (dem monoklonalen Antikörper, der von der Hybridom-Zelllinie mit der ATCC-Hinterlegungsnummer HB 11659 sekretiert wird) erkannt wird, zur Herstellung eines Medikaments zur Behandlung von B-Zellen-Proliferations-Erkrankungen.

2. Verfahren zum Abtöten von B-Zellen innerhalb einer Mischpopulation von Zellen in vitro, wobei das Verfahren das Kontaktieren der Mischpopulation von Zellen mit einer zytotoxischen Menge eines mehrwertigen Mittels umfasst, das mehr als ein CDIM-Epitop bindet.

3. Verfahren nach Anspruch 2, worin das mehrwertige Mittel ein Antikörper ist.

4. Verfahren nach Anspruch 3, worin der Antikörper ein monoklonaler Antikörper ist.

5. Verfahren nach Anspruch 4, worin der monoklonale Antikörper ein Human-Antikörper ist.

6. Verfahren nach Anspruch 4, worin der monoklonale Antikörper ein IgM ist.

7. Verfahren nach Anspruch 2, worin die B-Zellen neoplastisch sind.

8. Verfahren nach Anspruch 2, worin die Mischpopulation von Zellen hämopoetisch ist.

9. Verwendung nach Anspruch 1, worin die B-Zellen neoplastisch sind.

10. Verwendung nach Anspruch 1, worin die B-Zellen eine Autoimmunerkrankung vermitteln.

11. Verwendung nach Anspruch 1, worin das Mittel ein monoklonaler Antikörper ist.

12. Verwendung nach Anspruch 11, worin der Antikörper ein Human-IgM ist.

13. Verfahren zum Isolieren eines B-Zellen-Markermoleküls, worin der Marker **dadurch gekennzeichnet ist, dass** er ein CDIM-Epitop aufweist, auf B-Zellen-Oberflächen zugänglich ist und von MAb 216 (dem monoklonalen Antikörper, der von der Hybridom-Zelllinie mit der ATCC-Hinterlegungsnummer HB 11659 sekretiert wird) erkannt wird, wobei das Verfahren Folgendes umfasst:
das Kombinieren eines aus einer B-Zellen enthaltenden Zellpopulation hergestellten Lysats mit einem Antikörper, der sich an das CDIM-Epitop bindet, um einen Immunkomplex zu bilden;
das Abtrennen des Immunkomplexes von anderen Komponenten des Lysats; und
das Freisetzen des B-Zellen-Markermoleküls aus dem Komplex mit einer Verbindung, die um die Bindung an den Antikörper konkurriert.

14. Verfahren nach Anspruch 13, worin die kompetitive Bindungsverbindung Lacto-N-norhexaosylceramid ist.

15. Monoklonaler Antikörper, der von der Hybridom-Zelllinie mit der ATCC-Hinterlegungsnummer HB 11659 sekretiert wird.

16. Hybridom-Zellinie mit der ATCC-Hinterlegungsnummer HB 1 1659.

17. Verfahren nach Anspruch 5, worin der monoklonale Antikörper der von der Hybridom-Zelllinie mit der ATCC-Hinterlegungsnummer HB 11659 sekretierte monoklonale Antikörper ist.

18. Diagnoseset, das den von der Hybridom-Zelllinie mit der ATCC-Hinterlegungsnummer HB 11659 sekretierten monoklonalen Antikörper umfasst.

## Revendications

1. Utilisation d'un agent polyvalent qui se lie à plus d'un épitope CDIM, qui est un épitope reconnu par MAb 216 (l'anticorps monoclonal sécrété par la lignée de cellules d'hybridome ATCC dépôt No. HB 11659) pour la fabrication d'un médicament pour le traitement de maladies prolifératives à cellules B.

2. Méthode de destruction de cellules B dans une population mixte de cellules *in vitro*, ladite méthode comprenant l'étape consistant :
à mettre en contact ladite population de cellules mixtes avec une quantité cytotoxique d'un agent polyvalent qui se lie à plus d'un épitope CDIM.

3. Méthode selon la revendication 2, dans laquelle ledit agent polyvalent est un anticorps.

4. Méthode selon la revendication 3, dans laquelle ledit anticorps est un anticorps monoclonal.

5. Méthode selon la revendication 4, dans laquelle ledit anticorps monoclonal est un anticorps humain.

6. Méthode selon la revendication 4, dans laquelle ledit anticorps monoclonal est une IgM.

7. Méthode selon la revendication 2, dans laquelle lesdites cellules B sont néoplasiques.

8. Méthode selon la revendication 2, dans laquelle ladite population de cellules mixte est hématopoiétique.

9. Utilisation selon la revendication 1, dans laquelle lesdites B cellules B sont néoplasiques.

10. Utilisation selon la revendication 1, dans laquelle lesdites cellules B médient une maladie auto-immune.

11. Utilisation selon la revendication 1, dans laquelle ledit agent est un anticorps monoclonal.

12. Utilisation selon la revendication 11, dans laquelle ledit anticorps est une IgM humaine.

13. Méthode d'isolement d'une molécule marqueur des cellules B, dans laquelle le marqueur est **caractérisé en** ayant un épitope CDIM, est accessible en surface sur les cellules B, et est reconnu par MAb 216, (l'anticorps monoclonal sécrété par la lignée cellulaire d'hybridome ATCC dépôt HB 11659), ladite méthode comprenant les étapes consistant :
à combiner un lysat préparé à partir d'une population de cellules contenant des cellules B avec un anticorps se liant à l'épitope CDIM pour former un complexe immun ;
à séparer ledit complexe immun des autres composants dudit lysat ; et
à libérer la molécule marqueur des cellules B dudit complexe avec un composé qui entre en compétition pour la liaison audit anticorps.

14. Méthode selon la revendication 13, dans laquelle ledit composé qui entre en compétition pour la liaison est le lacto-N-norhexaosylcéramide.

15. Anticorps monoclonal sécrété par la lignée cellulaire d'hybridome ATCC dépôt No. HB 11659.

16. Lignée cellulaire d'hybridome dépôt ATCC No. HB 11659.

17. Méthode de la revendication 5, dans laquelle l'anticorps monoclonal est l'anticorps monoclonal sécrété par la lignée cellulaire d'hybridome ATCC dépôt No. HB 11659.

18. Kit de diagnostic comprenant l'anticorps monoclonal sécrété par la lignée cellulaire d'hybridome ATCC dépôt No. HB 11659.
